# EUROPEAN PATENT SPECIFICATION

(11) **EP 1 333 848 B1**
(45) Date of publication and mention of the grant of the patent: **05.04.2006**
(21) Application number: 01986262.2
(22) Date of filing: 05.10.2001
(51) Int. Cl.: A61K 36/47, A61K 127/00, A61K 31/70, A61P 31/14

(54) **PHYLLANTHUS-DERIVED COMPOUNDS FOR THE PREVENTION AND/OR TREATMENT OF DISEASES ASSOCIATED WITH A RETROVIRUS**
VERBINDUNGEN AUS PHYLLANTHUS ZUR PRÄVENTION UND/ODER BEHANDLUNG VON ERKRANKUNGEN IM ZUSAMMENHANG MIT EINEM RETROVIRUS
COMPOSES DERIVES DE PHYLLANTHUS UTILISES DANS LA PREVENTION ET/OU LE TRAITEMENT DES MALADIES ASSOCIEES A UN RETROVIRUS

(30) Priority: 06.10.2000 EP 00121852; 08.06.2001 EP 01114008
(43) Date of publication of application: 13.08.2003
(73) Proprietor: Phytrix AG, 80335 München (DE)
(72) Inventor: WAGNER, Ralf, 93049 Regensburg (DE); NOTKA, Frank, 93059 Regensburg (DE)
(74) Representative: Vossius & Partner
(86) International application number: PCT/EP2001/011524
(87) International publication number: WO 2002/028403

(56) References cited:
- WO-A-89/09059
- WO-A-90/04968
- DATABASE BIOSIS [Online] BIOSCIENCES INFORMATION SERVICE, PHILADELPHIA, PA, US; 1999 HOUGHTON P J ET AL: "The anti-HIV activity of an aqueous extract and polyphenolic compounds of Phyllanthus amarus." Database accession no. PREV199900538759 XP002191772 & JOURNAL OF PHARMACY AND PHARMACOLOGY, vol. 51, no. SUPPL., 1999, page 100 136th British Pharmaceutical Conference;Cardiff, Wales, UK; September 13-16, 1999 ISSN: 0022-3573
- DATABASE BIOSIS [Online] BIOSCIENCES INFORMATION SERVICE, PHILADELPHIA, PA, US; 1992 HAYAMIZU K ET AL: "RETROVIRAL REVERSE TRANSCRIPTASE-INHIBITORY ACTIVITY OF FLAVONOID AND TANNIN COMPOUNDS" Database accession no. PREV199294037003 XP002191773 & BULLETIN OF THE COLLEGE OF AGRICULTURE AND VETERINARY MEDICINE NIHON, no. 49, 1992, pages 35-41, 1992 ISSN: 0078-0839
- HOUGHTON P.J. ET AL JOURNAL OF PHARMACY AND PHARMACOLOGY vol. 51, no. SUPPL., 1999, pages 100 - 100, XP009031172

## Description

The present invention relates to the use a Phyllanthus extract or a fraction thereof or a gallotannin or a combination of gallotannins for the preparation of a pharmaceutical composition for the prevention or treatment of retrovirus related diseases associated with a nucleosidic inhibitor-resistant retrovirus and/or a non-nucleosidic inhibitor-resistant retrovirus. Preferably, the retrovirus is a human immunodeficiency virus (HIV). In accordance with the present invention, it is further preferred that said gallotannin or combination of gallotannins is or comprises corilagin and/or geraniin and/or another ellagitannin. Further preferred is that said Phyllanthus is Phyllanthus amarus.

Infection of retroviruses can cause severe illness and often results in death of humans. Retroviruses pathogenic for humans include Rous sarcoma virus, human T-cell leukemia virus (HTLVI and HTLVII) as well as human immunodeficiency virus (HIV). In particular, HIV is of major concern for health authorities due to its spread, its mode of infection and since a satisfactory cure has not been identified so far. Presently, treatment of HIV infected patients relies on a combination therapy that is expensive and may give rise to resistant HIV strains after prolonged progression of the therapy.

Phyllanthus extracts or components derived from Phyllanthus have been described as antiviral agents in the art. Thus, US-A 4,937,074 (Venkateswaran et al.) reports that Phyllanthus niruri extracts may inhibit reverse transcriptase (RT) activity of retroviruses such as HIV. The extract may be obtained by extracting whole plants with methanol or other conventional solvents. Uchiumi et al., Biochem. Biophys. Res. Commun. 220 (1996), 411-417 describe the use of tannic acids for the suppression of HIV promoter activity. Liu et al., Planta Med. 65 (1999), 43-46 describe that the variety of ellagitannins such as corilagin and geraniin have a strong inhibitory effect on HIV reverse transcriptase. Hirayama, US-A 5,159,060 describe the use of sulfated tannins for inhibiting reverse transcriptase of retroviruses.

The above reports all rely on the inhibition of viral spread or RT-activity from wild-type retroviruses. Such viruses are normally susceptible to nucleosidic inhibitors or non-nucleosidic inhibitors. After treatment with such inhibitors or combinations of such inhibitors, it has been observed that HIV may develop a resistance to therapy. Once the viruses show resistance against said inhibitors, present therapy approaches usually fail.

Accordingly, the technical problem underlying the present invention was to provide novel approaches for treating or preventing infections by retroviruses that are resistant to nucleosidic or non-nucleosidic inhibitors. The solution to said technical problem is provided by the embodiments characterized in the claims.

Accordingly, the present invention relates to the use of a Phyllanthus extract or a fraction thereof or a gallotannin or a combination of gallotannins for the preparation of a pharmaceutical composition for the prevention or treatment of retrovirus-related diseases associated with a nucleosidic inhibitor-resistant retrovirus and/or a non-nucleosidic inhibitor-resistant retrovirus.

The present invention also relates to the use of a Phyllanthus extract or a fraction thereof or a gallotannin or a combination of gallotannins for the preparation of a pharmaceutical composition for the inhibition of the replication of a nucleosidic inhibitor-resistant retrovirus and/or a non-nucleosidic inhibitor-resistant retrovirus.

The present invention furthermore relates to the use of a Phyllanthus extract or a fraction thereof or a gallotannin or a combination of gallotannins for the preparation of a pharmaceutical composition for the inhibition of the virus-uptake of a nucleosidic inhibitor-resistant retrovirus and/or a non-nucleosidic inhibitor-resistant retrovirus.

The pharmaceutical composition prepared in accordance with the present invention may further comprise a pharmaceutically acceptable carrier and/or diluent. Examples of suitable pharmaceutical carriers are well known in the art and include phosphate buffered saline solutions, water, emulsions, such as oil/water emulsions, various types of wetting agents, sterile solutions etc. Compositions comprising such carriers can be formulated by well known conventional methods. These pharmaceutical compositions can be administered to the subject at a suitable dose. Administration of the suitable compositions may be effected by different ways, e.g., by oral, intravenous, intraperitoneal, subcutaneous, intramuscular, topical, intradermal, intranasal or intrabronchial administration. The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. A typical dose can be, for example, in the range of 150 to 1200 mg; however, doses below or above this exemplary range are envisioned, especially considering the aforementioned factors. Generally, the regimen as a regular administration of the pharmaceutical composition should be in the range of 3 to 6 units per day. Progress can be monitored by periodic assessment. The compositions may be administered locally or systemically. Administration will generally be orally, in the above indicated dose range. Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like.
The term "retrovirus-related diseases associated with a nucleosidic inhibitor-resistant retrovirus and/or a non-nucleosidic inhibitor-resistant retrovirus" refers to diseases or disease states that are induced by or co-associated with such inhibitor-resistant retroviruses. For example, said diseases or disease states may have been induced by non-resistant retrovirus strains and controlled with a suitable treatment, but resistant strains may have been induced. Due to the pathogenic effects of said resistant retroviruses which may have previously been induced, the disease or disease state may deteriorate. Further, said diseases or disease states may have been induced by resistant retroviruses, following primary infection with resistant retroviruses. It is important to note that the term "resistant" also includes partial resistance, typically at least 10 % resistance.

The term "virus-uptake of a nucleosidic inhibitor-resistant retrovirus and/or a non-nucleosidic inhibitor-resistant retrovirus" refers to entry of a retrovirus into its target cell. It has been shown that a retrovirus enters its target cell by a process comprising at least three consecutive steps. The first step involves binding of the retrovirus to molecules on the surface of its target cell. The HIV, for example, binds to its target cells via the viral glycoprotein gp41 and gp120. In a further step, the virus is taken up into the target cell by a mechanism involving endocytosis of the viral particle into said target cell. In the following step, the virus is uncoated and its RNA genetic material and the virally encoded reverse transcriptase enzyme are released into the cytoplasm. The reverse transcriptase starts the replication cycle of the retrovirus by transcribing the RNA genetic material into the DNA provirus which is subsequently integrated into the genome of the target cell. Target cells for the retroviruses are cells which are capable of binding the retrovirus via cell surface molecules. For example, said target cells are CD4 positive cells in the case of the HIV-1.

In accordance with the present invention, it has been found that Phyllanthus extracts effectively inhibit the replication of retroviruses that are resistant to nucleosidic inhibitors and/or non-nucleosidic inhibitors by the inhibition of the infection of CD4 T-cells. Moreover, it has been found that in addition to virus replication, the virus-uptake by the target cells was also significantly inhibited. Surprisingly, the aforesaid extracts, fractions and gallotannins were found to be as effective or were more effective against several NRTI (nucleosidic reverse transcriptase inhibitors) strains, several NNRTI (non-nucleosidic reverse transcriptase inhibitors) strains and one HIV-2 strain as compared to the wild-type HIV-1 strain demonstrating an unusual broad protective potential. Advantageously, the Phyllanthus extracts or fractions thereof or gallotannins or a combination of gallotannins are capable of inhibiting simultaneously at least two key steps of the life cycle of a retrovirus, namely uptake and reverse transcription in vitro.

In a preferred embodiment of the use of the invention, said retrovirus is an HIV, a Rous sarcoma virus or a HTLV I or II.

The HIV may be an HIV-1 or HIV-2. Particularly preferred is that said HIV is an HIV-1.

The Phyllanthus extract may be prepared according to conventional methods well-known in the art; see, for example, Liu et al.,loc. cit. Hirayama et al., loc. cit., Venkateswaran et al., loc. cit. Preferably, said extract is a water extract, an alcohol extract, a water-alcohol extract, an hexane extract or a CO₂ extract.

It is further preferred that the compounds of Phyllanthus used as active ingredients are not destroyed during the extraction process. Such extraction processes are known in the art and have been referred to herein above by reference.

It is most preferred that said alcohol is methanol or ethanol.

It is further preferred in accordance with the invention that said fraction is a protein fraction or a gallotannin fraction. Protein fractions may also be obtained according to conventional protocols. Appropriate protocols for obtaining gallotannin fractions are also well-known in the art; see, for example, Liu et al., loc. cit. For example, 100 g of freshly powdered drug maybe heated with 1000 ml distilled methanol for two hours in a round flask. After cooling, the solution is filtered through cotton wool. The solid sediment is subsequently heated twice with 500 ml methanol followed by decanting after cooling. The combined methanolic extracts are subsequently dried in a rotary evaporator. The dry sediment may be resuspended in 200 ml distilled water and subjected to ultrasonic treatment for three minutes. The suspension thus obtained may be three times extracted with 100 ml dichloromethane. The combined dichloromethane phases are subsequently extracted with 100 ml distilled water. The aqueous phases are combined, about half of the liquid removed in a rotary evaporator and the product of this process is subsequently freeze-dried. A typical amount of gallotannin fraction obtained is 10.5 g.

It is further preferred in accordance with the present invention that said gallotannin is or said gallotannin fraction comprises corilagin.

Also preferred is that said gallotannin is or said gallotannin fraction comprises geraniin.

Further preferred is that said gallotannin is or said gallotannin fraction comprises ellagic acid.

Additionally preferred is that said gallotannin is or said gallotannin fraction comprises brevifolin carboxylic acid.

In accordance with the present invention, it is further preferred that said gallotannin is or said gallotannin fraction comprises another ellagitannin. Examples of further ellagitannins are chebulagic acid, elaeocarpusin, mallotusinin, phyllantusiin and 1-galloyl-3, 6-hexahydrodiphenoyl-4-0-brevifolincarboxyl-β-D-glucopyranose.

In another preferred embodiment of the use of the invention, said combination of gallotannins or said gallotannin fraction comprises at least two such as three, four or five of corilagin, geraniin, ellagic acid and brevifolin carboxylic acid and optionally another ellagitannin. The relative amounts of coraligin, geraniin, ellagic acid and brevifolin carboxylic acid and optionally said other ellagitannin may vary in the pharmaceutical composition. It is preferred that at least three different ellagitannins including corilagin and geraniin are present in said pharmaceutical composition.

In another preferred embodiment of the use of the present invention, said gallotannins are formulated into said pharmaceutical composition in a final concentration of 0.1 to 99.9 %, preferably 1 to 95 %. This holds in particular true for oral administration.

Also preferred is that said extract or said fraction is present in a final concentration of 0.1 to 99.9 %, preferably 1 to 95 % in said pharmaceutical composition. This holds in particular true for oral administration.
Wherein said Phyllanthus may belong to a variety of different Phyllanthus species (all which are comprised by the present invention), it is preferred that said Phyllanthus is Phyllanthus amarus.

The present invention also relates to the use in the treatment of patients affected by a disease caused by or associated with a retrovirus that is resistant to nucleosidic or non-nucleosidic reverse transcriptase inhibitors comprising administration of a suitable amount of a Phyllanthus extract or a fraction thereof or a gallotannin or a combination of gallotannins to said patient. Preferably, said retrovirus is an HIV, more preferably an HIV-1. Also preferred is that said gallotannin is corilagin or geraniin or another ellagitannin or a combination thereof. Further preferred embodiments of the method of treatment of the present invention have been discussed in connection with the use of the invention and are equally applicable.

Finally, the present invention relates to methods of inhibiting the replication of retroviruses wherein said retrovirus is resistant to nucleosidic or non-nucleosidic reverse transcriptase inhibitors comprising administering a suitable amount of a Phyllanthus extract or a fraction thereof or a gallotannin or a combination of gallotannins to said patient infected by said retrovirus. Further preferred embodiments of the method of inhibition of the present invention have been discussed in connection with the use of the invention and are equally applicable.

The Figures show:
Figure 1: graphically represents percent inhibition (ordinate) of human immunodeficiency virus type 1 reverse transcriptase determined *in vitro* as a function of concentration of corilagin and geraniin purified from *P. amarus* (*µ*g/ml) (abscissa).
Figure 2: graphically represents percent inhibition (ordinate) of human immunodeficiency virus type 1 replication in MT4 cells determined *in vitro* as a function of concentration of geraniin purified from *P. amarus* (*µ*g/ml) (abscissa).
Figure 3: graphically represents percent inhibition (ordinate) of replication of a wild type HX10 and a nucleosidic reverse transcriptase inhibitor resistant variant ARP145 of human immunodeficiency virus type 1 in MT4 cells determined *in vitro* at different concentrations of geraniin purified from *P. amarus* (*µ*g/ml) (abscissa).
Figure 4: graphically represents percent inhibition (ordinate) of replication of a wild type HX10 and a nucleosidic reverse transcriptase inhibitor resistant variant ARP145 of human immunodeficiency virus type 1, and a wild type human immunodeficiency virus type 2 (HIV-2 rod) in a HeLa CD4⁺ reporter cell line determined *in vitro* as a function of concentration of *P. amarus* CMI-111 extract (Example 7) (*µ*g/ml) (abscissa).
Figure 5: graphically represents percent inhibition (ordinate) of human immunodeficiency virus 1 reverse transcriptase determined *in vitro* as a function of concentration of *P. amarus* CMI 111 extract (Example 7) (*µ*g/ml) (abscissa).
Figure 6: HPLC fingerprint of the Ph. amarus dry extract.
Figure 7: HPLC fingerprint of the Ph. amarus dry extract + brevifolin carboxylic acid (4a).
Figure 8: graphically represents percent reduction of intracellular HIV-1 p24 concentration (=inhibition of virus internalization) (ordinate) in a HeLa CD4⁺ reporter cell line infected with HIV-1 determined *in vitro* in the presence of geraniin at different concentrations (*µ*g/ml) (abscissa). Percent reduction for heparin sulfate (Hep-S) at a concentration of 100 *µ*g/ml and for an α-HIV-1 gp120 monoclonal antibody (final dilution 1:40) are also depicted.
Figure 9: graphically represents percent reduction of intracellular HIV-1 p24 concentration (=inhibition of virus internalization) (ordinate) in a HeLa CD4⁺ reporter cell line infected with HIV-1 determined *in vitro* in the presence of CMI-111 at different concentrations (*µ*g/ml) (abscissa). Percent reduction for heparin sulfate (Hep-S) at a concentration of 100 *µ*g/ml and for an α-HIV-1 gp120 monoclonal antibody (final dilution 1:40) are also depicted.
Figure 10: graphically represents percent reduction of HIV-1 gp120 binding to immobilized CD4 (=inhibition of virus receptor binding) (ordinate) determined in vitro in the presence of geraniin at different concentrations (*µ*g/ml) (abscissa).
Figure 11 graphically represents percent reduction of HIV-1 gp120 binding to immobilized CD4 (=inhibition of virus receptor binding) (ordinate) determined in vitro in the presence of CMI-111 at different concentrations (*µ*g/ml) (abscissa).

The Examples illustrate the invention.

### Example 1

### Anti HIV-1 activity of gallotannins from P. amarus in infection assays

HIV-1 (HX10, Ratner L., et al. (1987; AIDS research and retroviruses 3, 57-69 or HBIO, Accession No. 15654, Wong-Staal F et al. (1985), Nature 313, 277-284) was prepared from a conditioned culture fluid of a MT4 cell line (Miyoshi I et al. (1982), Gann Monograph on Cancer Res 28:219; Pauwels R et al. (1987), J. Virol. Methods 16: 171). The culture fluid was clarified of cells by low speed centrifugation (900xg) and an equal volume of fetal calf serum (FCS) was added before storage at -80°C. Inhibition of virus infection by *P. amarus* gallotannin extract and purified gallotannins corilagin and geraniin was evaluated using a HeLa-CD4-LTR-β-gal reporter cell line *(Multinuclear activation of galactosidase indicator (MAGI) cells,* Kimpton J, Emerman M. (1992), J Virol 66(4):2232-2239). In brief, 1,5 x 10⁴ MAGI cells/well were plated in 48 well culture plates and grown overnight. The next day cells were incubated with 70 *µ*l of HIV-1 stock in 100 *µ*l in the presence of gallotannin extract or corilagin or geraniin at concentrations of 10 *µ*g/ml, 5 *µ*g/ml, 2.5 *µ*g/ml or 1.25 *µ*g/ml, or PBS for 2 hours in a humidified atmosphere. Then 200 *µ*l medium with or without drugs was added and cells were incubated for 2 days. Infected cells were detected by 5-bromo-4-chloro-3-inolyl-galactopyranoside (X-Gal) staining of cells expressing an endogenous β-galoctidase as a consequence of HIV infection. Cells were fixed by incubation with 0.2% glutaraldehyde and 1% formaldehyde in PBS for 5 min. Fixed cells were washed with PBS twice and overlaid with the staining solution (4 mM K-Ferricyanide, 4 mM K-Ferrocyanide, 2 mM MgCl₂ and 0.4 mg/ml X-Gal) for 30 min at 37°C. The number of blue cells was determined by microscopically observation (for details see Kimpton J, Emerman M. (1992). *J Virol* 66(4): 2232-9). Antiviral activity was calculated from the ratio of blue stained cells with and without extract and is given as percent inhibition. Tests were performed in triplicates. The inhibition of infection is reported in table 1. As can be seen from table 1 gallotannins inhibit HIV-1 replication in a dose dependent manner.

**Table 1. Effects of P. amarus gallotannins in an HIV-1 infection reporter assay**

| | **% Inhibition** | | | |
|---|---|---|---|---|
| | **Concentration** | | | |
| **Inhibitor** | **10 *µ*g/ml** | **5 *µ*g/ml** | **2.5 *µ*g/ml** | **1.25 *µ*g/ml** |
| Gallotannin extract | 94±1 | 81 ±1 1 | 63 ± 6 | 23 ±1 |
| Corilagin | 85 ± 7 | 73 ± 9 | 38 ±13 | 9 ±16 |
| Geraniin | 98 ± 3 | 93 ± 5 | 80 ± 4 | 66 ± 8 |

### Example 2

### Inhibition of HIV-1 reverse transcriptase in vitro using gallotannins of P. amarus

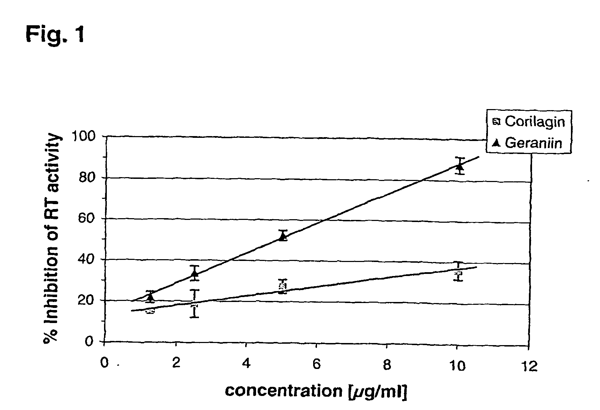

The inhibitory effect of *P. amarus* gallotannins corilagin and geraniin on recombinant HIV-1 reverse transcriptase (RT) was determined by a commercially available RT assay (Screen RTA, Retro-Tech GmbH, Unterschleißheim, Germany), that utilizes the incorporation of biotinylated nucleotides in DNA as a measure of reverse transcriptase. The recombinant RT-reagent (5 mU) was incubated with corilagin or geraniin at concentrations of 10 *µ*g/ml, 5 *µ*g/ml, 2.5 *µ*g/ml or 1.25 *µ*g/ml, or PBS for 2 h and RT activity was measured according to the manufacturer's protocol. The inhibition of recombinant HIV-1 RT by *P. amarus* gallotannins is graphically represented in Figure 1. From Figure 1 it can be seen that the increase in inhibition is linear with increasing concentration of the gallotannins up to 10 *µ*g/ml at which point the inhibition is >80% for geraniin and >35% for corilagin.

### Example 3

### Anti HIV-1 activity of geraniin in infection assays

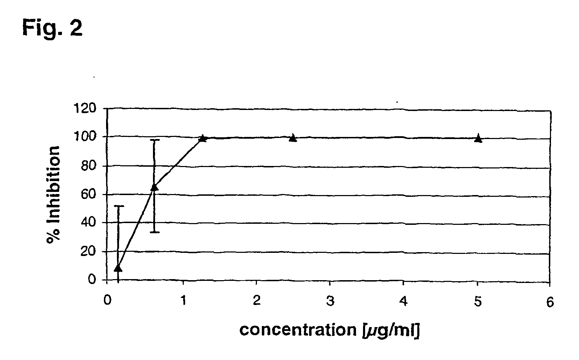

The HIV-1 virus stock, described in Example 1, was used to asses the inhibitory effect of by *P. amarus* geraniin on the infection of CD4⁺ lymphoide cells (MT4). The 50% tissue culture infectious dose (TCID₅₀) was determined by infection of MT4 cells with serial dilutions of the virus stock and subsequent quantification of viral p24 content in the cell fluid (HIV-1 p24 core profile ELISA, NEN, Zaventem, Belgium). MT4 cells were infected with ca. 100 TCID₅₀ in the presence of *P. amarus* purified geraniin at concentrations of 5 *µ*g/ml, 2.5 *µ*g/ml, 1.25 *µ*g/ml, 0.63 *µ*g/ml and 0.31 *µ*g/ml. It was previously determined that geraniin was non-toxic at these concentrations. The MT4 target cells were incubated for 7 days in the presence of virus and inhibitor, and fresh medium (containing drugs) was added every other day. Infections were monitored by quantification of p24 content in the cell fluid. Samples without detectable p24 contents (compared to the reference inhibitors and uninfected negative controls) were regarded completely protected from infection. Otherwise the content of p24 in the fluid was negatively correlated to protection. Each experiment was performed in triplicates. Figure 2 graphically depicts the results of two independent tests (means, error bars reflect standard deviation). From Figure 2 it can be seen that increasing geraniin concentration results in increased inhibition with complete inhibition at a concentration of 1.25 *µ*g/ml.

### Example 4

### Anti HIV-1 activity of geraniin on a RT inhibitor resistant HIV-1 strain in infection assays

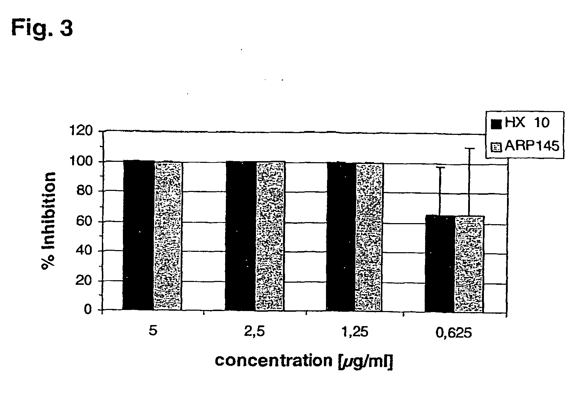

Essentially the same experiment as described in Example 3 was used to determine the inhibitory effect of geraniin on a nucleosidic RT inhibitor (NRTI) resistant HIV-1 strain. The strain termed ARP145 (Tisdale et al. (1993), Proc Nat Acad Sci (USA) 90(12): 5653-5656) was obtained from the NIBSC (MRC) and is phenotypically characterized by a 3TC/FTC resistance. MT4 cells were infected with ca. 100 TCID₅₀ of either HX10 or ARP145 in the presence of *P. amarus* purified geraniin at concentrations of 5 *µ*g/ml, 2.5 *µ*g/ml, 1.25 *µ*g/ml and 0.63 *µ*g/ml. The inhibition of the resistant strain and of the wild type parental strain (HX10) is graphically represented in Figure 3. No significant difference was observed in the inhibition of wild type or NRTI resistant strain by geraniin.

### Example 5

### Anti HIV-1 activity of Phyllanthus amarus extract on a RT inhibitor resistant HIV-1 and a HIV-2 strain in infection assays

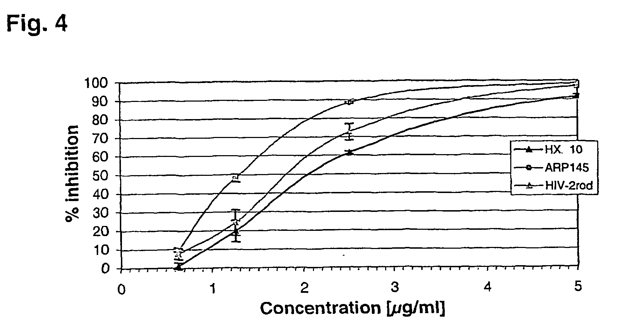

Essentially the same experiment as described in Example 1 was used to determine the inhibitory effect of a Phyllanthus extract prepared as described in Example 7 on a HIV-2 strain *(ROD,* Clavel F et al. (1986), Science 233: 343; Guyader M et al. (1987), Nature 326: 662) and a nucleosidic RT inhibitor (NRTI) resistant HIV-1 strain (ARP145). MAGI cells were infected with 70 *µ*l of the corresponding virus stock in the presence of the extract at concentrations of 5 *µ*g/ml, 2.5 *µ*g/ml, 1.25 *µ*g/ml and 0.63 *µ*g/ml (in phosphate buffered saline). The inhibition of the HIV-2 strain, of the NRTI resistant strain and of the wild type parental strain (HX10) is graphically represented in Figure 4. No decrease of inhibition was observed in the inhibition of the HIV-2 or NRTI resistant HIV-1 strain compared to the HIV-1 wild type strain by the extract. To the contrary, inhibition of HIV-2 was enhanced. Even more enhanced was the inhibition of the NRTI resistant strain.

### Example 6

### Inhibition of HIV-1 reverse transcriptase in vitro using Phyllanthus amarus extract

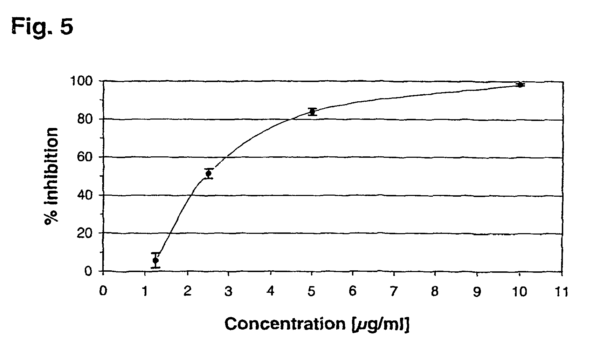

The inhibitory effect of *P. amarus* extract (Example 7) on recombinant HIV-1 reverse transcriptase (RT) was determined essentially in the same way as described in Example 2. However, in this experiment a different RT assay was used (Reverse Transcriptase Assay, non-radioactive, Roche Diagnostics GmbH, Mannheim, Germany). The recombinant RT-reagent (2.5 mU) was incubated with the extract at concentrations of 10 *µ*g/ml, 5 *µ*g/ml, 2.5 *µ*g/ml or 1.25 *µ*g/ml, or PBS for 1 h and RT activity was measured according to the manufacturer's protocol. The inhibition of recombinant HIV-1 RT by the extract is graphically represented in Figure 5. From Figure 5 it can be seen that inhibition on the recombinant HIV-1 RT is dose dependent. At an extract concentration of 10 *µ*g/ml inhibition is nearly complete (>98%).

### Example 7

### Preparation of Phyllanthus amarus extract

Whole uncut dry leaves were extracted in steel vessels with a drug solvent ratio 1:10 (+/-3) of ethanol 50 % (v/v) for 1 to 3 hours at 30-40 °C. The drug residues were separated and then washed with 1 to 3 parts water. The ethanolic eluat and the water were combined. Then disodiumhydrogencitrate (1-3 % m/m) was added and the liquid subsequently filtrated through a membrane. Powdered Sterculia (presolved in ethanol/water) was added to the extract liquid (0.5-5 % m/m final concentration). Following the liquid was evaporated under reduced pressure (approx. 300 mbar decreasing to 20 mbar), evaporation temperature 30 to 60 °C (+/-5 °C) until the dry material content of 20-40 % dry mass (m/m) was reached. Subsequently the soft extract was mixed with maltodextrin (28-39 % m/m) until a homogenous suspension occurred. The mixture was processed through a short-heating unit for reduction of microbial contamination at a temperature of 80-120 °C and a duration of 20-40 sec. The mixture was thoroughly dried until the water content is less then 5 %. During the drying process the outlet temperature of the drying unit did not exceed 90 °C (+/- 5 %) (= "product temp."). Silica (0.5-3 % m/m) was added during the drying process and after the drying process. The dried product was mixed and subsequently sieved.

### Example 8: Purification of compounds from Phyllanthus amarus

### 1. Isolation Procedure

Starting material for the isolation of the brevifolin carboxylic acid was the extract as prepared in Example 7.
The dry extract was dissolved in 50 % aqueous ethanol and centrifuged to remove insoluble material and fines. The clear supernatant was applied to a Sephadex LH20 column run in 20 % aqueous methanol. The purified fraction X from the column chromatography was concentrated in a rotary evaporator and freeze dried.

### 2. Identification procedure

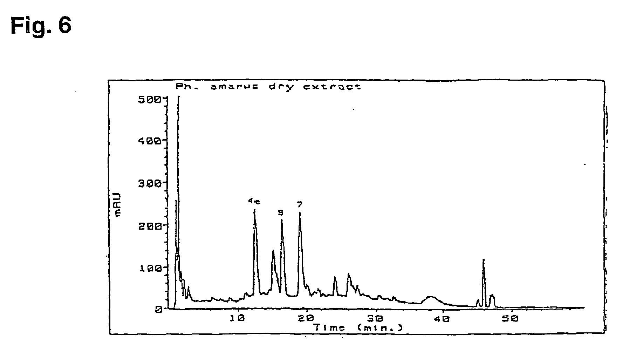

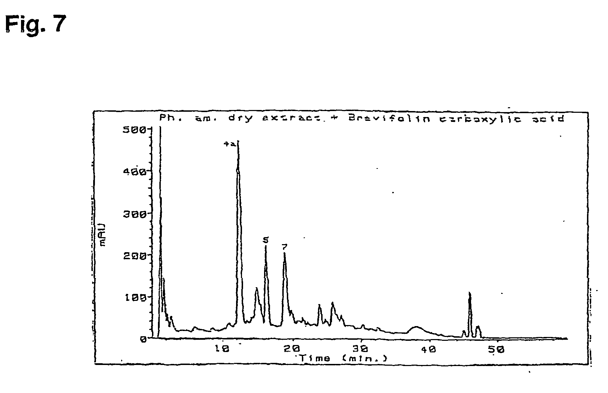

The purified compound X was identified by UV-spectroscopy and ¹³C-NMR. The main peaks in UV were at 278, 350 and 362 nm in complete accordance to the literature data (M. A. M. Nawwar et al. Phytochemistry 36(3), 793-798, 1994). Our NMR data confirm the existence of 13 C-atoms and the structure of brevifolin carboxylic acid as described in Phytochemistry (see above). The extract was subjected to HPLC analysis (Fig. 6). Additional evidence was given by spiking the original HPLC fingerprint with an authentic sample of brevifolin carboxylic acid from Prof. T. Yoshida, Okayama University, Japan (see Fig. 7).

### 3. Summary

From the gallotannin fraction of Ph. amarus another major gallotannin compound has been isolated and identified as brevifolin carboxylic acid. This compound can be obtained as a degradation product of geraniin.
Herewith four major compounds of Ph. amarus were identified:
- geraniin (7)
- corilagin (5)
- ellagic acid
- brevifolin carboxylic acid (4a)

### Example 9

### Anti HIV-1 activity of geraniin on a HIV-2 strain and a selection of RT inhibitor resistant HIV-1 strains in infection assays

Essentially the same experiment as described in Example 1 was used to determine the inhibitory effect of geraniin on a HIV-2 *(rod)* strain and a selection of nucleosidic RT inhibitor (NRTI) resistant HIV-1 strains (ARP141, ARP145 and ARP146) as well as non-nucleosidic RT inhibitor (NNRTI) resistant HIV-1 strains (ARP1010, ARP1011 and ARP1014). MAGI cells were infected with 70 *µ*l of the corresponding virus stocks in the presence of geraniin at concentrations of 2.5 *µ*g/ml, 1.25 *µ*g/ml and 0.63 *µ*g/ml. The EC₅₀ values of the HIV-2 strain, the RT resistant strains and the wild type parental strain (HX10) were calculated and are listed in table 2. No significant change of EC₅₀ values was observed in the inhibition of the HIV-2 or RT resistant HIV-1 strain compared to the HIV-1 wild type strains by geraniin.

**Table 2. Effect of geraniin on wild type and RTI-resistant HIV strains**

| | wild type | | NRTI | | | NNRTI | | |
|---|---|---|---|---|---|---|---|---|
| Strain | HX10 | HIV-2rod | ARP141 | ARP145 | ARP146 | ARP1010 | ARP1011 | ARP1014 |
| EC₅₀ [*µ*g/ml] | 0.74 | 0.83 | 0.83 | 0.95 | 0.71 | 0.83 | 0.89 | 1.10 |

### Example 10

### Inhibition of virus-uptake by geraniin

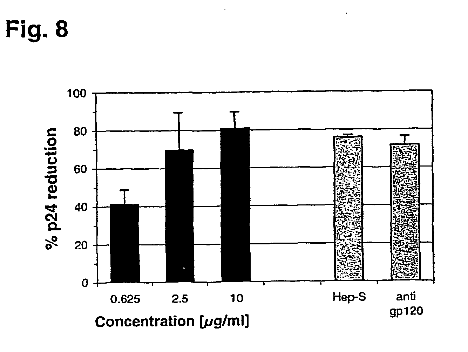

Inhibition of virus entry by geraniin was monitored using an assay described recently (Saphire et *al.* (1999) *EMBO Journal* 18: 6771-6785) with slight modifications. In brief, MAGI cells were preincubated at 4°C for 60 min (in 200 *µ*l), virus and inhibitor (in a volume of 100 *µ*l) were added, the cells were incubated for 30 min at 4°C and finally shifted to 37°C for two hours. The cells were then washed with 500 *µ*l PBS five times to remove unbound virus, trypsinized for 5 min to remove all attached but not internalized virus washed again with 500 µl PBS and finally lysed with 100 *µ*l PBS containing 0.5% NP-40. The HIV-1 p24 content of cell lysates was determined using a p24 sandwich ELISA (NEN). Heparin sulfate (Hep-S) at a concentration of 100 *µ*g/ml and an α-HIV-1 gp120 monoclonal antibody (final dilution 1:40) served as controls. Geraniin was tested for prevention of wild type HIV-1 uptake at final concentrations of 10 *µ*g/ml, 2.5 *µ*g/ml and 0.625 *µ*g/ml, the results are depicted in Figure 8. As can be seen, the internalization of virus was blocked by geraniin in a dose dependent manner.

### Example 11

### Anti HIV-1 activity of P. amarus extract (Example 7) also referred to as CMI-111 on a selection of RT inhibitor resistant HIV-1 strains in infection assays

Essentially the same experiment as described in Example 1 was used to determine the inhibitory effect of *P. amarus* extract on additional nucleosidic RT inhibitor (NRTI) resistant HIV-1 strains (ARP141 and ARP146) as well as non-nucleosidic RT inhibitor (NNRTI) resistant HIV-1 strains (ARP1010, ARP1011 and ARP1014). MAGI cells were infected with 70 *µ*l of the corresponding virus stocks in the presence of P. amarus extract at concentrations of 10 *µ*g/ml, 5 *µ*g/ml, 2.5 *µ*g/ml, 1.25 *µ*g/ml and 0.63 *µ*g/ml. The EC₅₀ values of the HIV-2 strain, the RT resistant strains and the wild type parental strain (HX10) were calculated and are listed in table 3. No. significant change of EC₅₀ values was observed in the inhibition of the HIV-2 or RT resistant HIV-1 strains compared to the HIV-1 wild type strain by CMI-111. To the contrary, inhibition of HIV-2 was enhanced. Even more enhanced was the inhibition of the RTI resistant strains.

**Table 3. Effect of CMI-111 on wild type and RTI-resistant HIV strains**

| | wild type | | NRTI | | NNRTI | | |
|---|---|---|---|---|---|---|---|
| Strain | HX10 | HIV-2rod | ARP141 | ARP146 | ARP1010 | ARP1011 | ARP1014 |
| EC₅₀ [*µ*g/ml] | 1.92 | 1.38 | 1.02 | 1.25 | 1.19 | 0.98 | 1.25 |

### Example 12

### Inhibition of virus-uptake by P. amarus extract (Example 7)

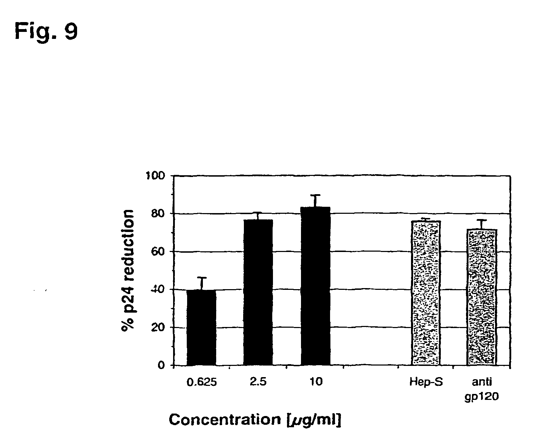

Inhibition of virus entry by P. amarus extract CMI-111 (Example 7)was determined essentially as described in Example 10. CMI-111 was tested for prevention of wild type HIV-1 uptake at final concentrations of 10 *µ*g/ml, 2.5 *µ*g/m) and 0.625 *µ*g/ml, the results are depicted in Figure 9. As can be seen, the internalization of virus was blocked by CMI-111 in a dose dependent manner.

### Example 13

### Inhibition of HIV-1 gp120 binding to CD4 by geraniin

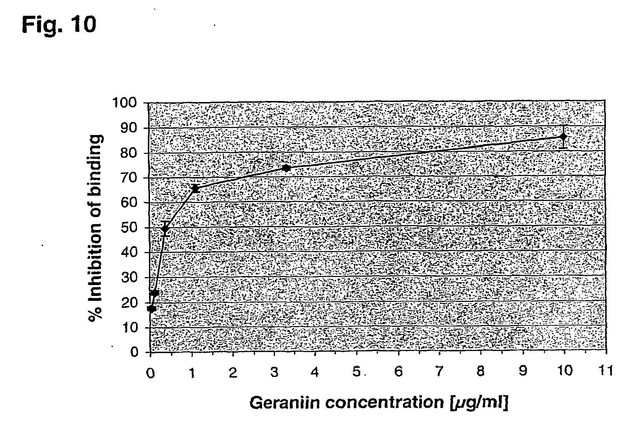

Inhibition of viral envelope glycoprotein gp120 binding to its cellular receptor CD4 by geraniin was monitored using immobilized recombinant CD4 and recombinant HIV-1 gp120 in an ELISA-like assay format. In brief, ELISA plates were coated with 100 ng/well CD4 (in 0.1 M carbonate buffer, pH 9.5) for 12 h at 4°C. Plates were washed 5 times with 200 *µ*l washing buffer (PBS, 0.05% Tween 20) and blocked with 200 *µ*l blocking buffer (PBS, 0.1% gelantine) for 1 h. Wells were incubated with 100 ng recombinant gp120 and dilutions of test samples (10% final concentration, in blocking buffer) for 1 h and washed 5 times. Wells were further incubated with 100 *µ*l mouse anti-gp120-V3 monoclonal antibody for 1 h at 37°C, washed 5 times and incubated with 100 *µ*l anti- mouse HRP-conjugated monoclonal antibody for 1 h at 37°C and washed 10 times. Finally, wells were incubated with 100 *µ*l substrate (OPD) for 15 min, the reaction was stopped with 100 *µ*l 1 N H2SO4 and the plate was read in an ELISA reader (492 nm). Geraniin was tested at final concentrations of 10 *µ*g/ml, 3.33 *µ*g/ml, 1.11 *µ*g/ml, 0.37 *µ*g/ml, 1.123 *µ*g/ml and 0.041 *µ*g/ml, in duplicates. The results of this representative experiment are depicted in figure 10. As can be seen, the binding of gp120 to CD4 was blocked by geraniin in a dose dependent manner. An average IC50 value of 0.48 ± 0.05 *µ*g/ml was calculated from three independent experiments.

### Example 14

### Inhibition of HIV-1 gp120 binding to CD4 by P. amarus extract

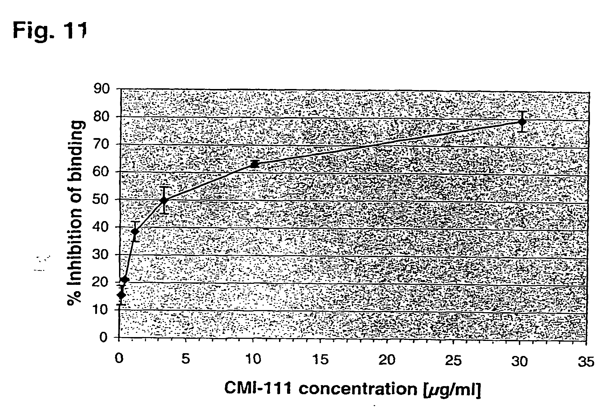

Inhibition of viral envelope glycoprotein gp120 binding to CD4 by P. amarus extract CMI-111 was determined essentially as described in example 13. CMI-111 was tested at final concentrations of 30 *µ*g/ml, 10 *µ*g/ml, 3.33 *µ*g/ml, 1.11 *µ*g/ml, 0.37 *µ*g/ml and 1.123 *µ*g/ml, in duplicates. The results are depicted in figure 11. As can be seen, the binding of gp120 to CD4 was blocked by CMI-111 in a dose dependent manner. An average IC50 value of 2.65 ± 0.44 *µ*g/ml was calculated from three independent experiments.

## Claims

1. Use of a Phyllanthus extract or a fraction thereof or a gallotannin or a combination of gallotannins for the preparation of a pharmaceutical composition for the prevention or treatment of retrovirus-related diseases associated with a nucleosidic inhibitor-resistant retrovirus and/or a non-nucleosidic inhibitor-resistant retrovirus.

2. The use of claim 1, wherein the diseases are treated by inhibiting the replication of a nucleosidic inhibitor-resistant retrovirus and/or a non-nucleosidic inhibitor-resistant retrovirus.

3. The use of claim 1, wherein the diseases are treated by inhibiting the virus-uptake of a nucleosidic inhibitor-resistant retrovirus and/or a non-nucleosidic inhibitor-resistant retrovirus.

4. The use of any one of claims 1 to 3, wherein said retrovirus is an HIV.

5. The use of claim 4, wherein said HIV is an HIV-1.

6. The use of any one of claims 1 to 3, wherein said extract is a water extract, an alcohol extract, a water/alcohol extract, a hexane extract or a CO2 extract.

7. The use of claim 6, wherein said alcohol is methanol or ethanol.

8. The use of any one of claims 1 to 5, wherein said fraction is a protein fraction or a gallotannin fraction.

9. The use of any one of claims 1 to 5 or 8, wherein said gallotannin is or said gallotannin fraction comprises corilagin.

10. The use of any one of claims 1 to or 8, wherein said gallotannin is or said gallotannin fraction comprises geraniin.

11. The use of any one of claims 1 to 5 or 8, wherein said gallotannin is or said gallotannin fraction comprises ellagic acid.

12. The use of any one of claims 1 to 5 or 8, wherein said gallotannin is or said gallotannin fraction comprises brevifolin carboxylic acid.

13. The use of any one of claims 1 to 5 or 8, wherein said gallotannin is or said gallotannin fraction comprises an ellagitannin different from corilagin, geraniin, ellagic acid and brevifolin carboxylic acid.

14. The use of claim 1 to 5 or 8, wherein said combination of gallotannins or said gallotannin fraction comprises at least two of corilagin, geraniin, ellagic acid and brevifolin carboxylic acid and optionally said ellagitannin different from corilagin and geraniin.

15. The use of any one of claims 1 to 14, wherein said gallotannins are formulated into said pharmaceutical composition in a concentration of 0.1 to 99.9 %.

16. The use of any one of claims 1 to 15, wherein said extract or said fraction is present in a concentration of 0.1 to 99.9 % in said pharmaceutical composition.

17. The use of any one of claims 1 to 16, wherein said Phyllanthus is Phyllanthus amarus.

## Patentansprüche

1. Verwendung eines Phyllanthusextrakts oder einer Fraktion davon oder eines Gallotannins oder einer Kombination von Gallotanninen für die Herstellung eines Arzneimittels zur Vorbeugung oder Behandlung von Retrovirus-assoziierten Krankheiten, die mit einem Nucleosidinhibitor-resistenten Retrovirus und/oder einem Nicht-Nucleosidinhibitor-resistenten Retrovirus in Verbindung stehen.

2. Verwendung nach Anspruch 1, wobei die Krankheiten behandelt werden, indem die Replikation eines Nucleosidinhibitor-resistenten Retrovirus und/oder eines Nicht-Nucleosidinhibitor-resistenten Retrovirus inhibiert wird.

3. Verwendung nach Anspruch 1, wobei die Krankheiten behandelt werden, indem die Virusaufnahme eines Nucleosidinhibitor-resistenten Retrovirus und/oder eines Nicht-Nucleosidinhibitor-resistenten Retrovirus inhibiert wird.

4. Verwendung nach einem der Ansprüche 1 bis 3, wobei das Retrovirus HIV ist.

5. Verwendung nach Anspruch 4, wobei das HIV ein HIV-1 ist.

6. Verwendung nach einem der Ansprüche 1 bis 3, wobei der Extrakt ein Wasserextrakt, ein Alkoholextrakt, ein Wasser/Alkohol-Extrakt, ein Hexanextrakt oder ein CO₂₋Extrakt ist.

7. Verwendung nach Anspruch 6, wobei der Alkohol Methanol oder Ethanol ist.

8. Verwendung nach einem der Ansprüche 1 bis 5, wobei die Fraktion eine Proteinfraktion oder eine Gallotanninfraktion ist.

9. Verwendung nach einem der Ansprüche 1 bis 5 oder 8, wobei das Gallotannin Corilagin ist oder die Gallotanninfraktion es umfasst.

10. Verwendung nach einem der Ansprüche 1 bis 5 oder 8, wobei das Gallotannin Geraniin ist oder die Gallotanninfraktion es umfasst.

11. Verwendung nach einem der Ansprüche 1 bis 5 oder 8, wobei das Gallotannin Ellagsäure ist oder die Gallotanninfraktion sie umfasst.

12. Verwendung nach einem der Ansprüche 1 bis 5 oder 8, wobei das Gallotannin Brevifolincarbonsäure ist oder die Gallotanninfraktion sie umfasst.

13. Verwendung nach einem der Ansprüche 1 bis 5 oder 8, wobei das Gallotannin von Corilagin, Geraniin, Ellagsäure und Brevifolincarbonsäure verschiedenes Ellagitannin ist oder die Gallotanninfraktion diese umfasst.

14. Verwendung nach einem der Ansprüche 1 bis 5 oder 8, wobei die Kombination von Gallotanninen oder die Gallotanninfraktion zumindest zwei von Corilagin, Geraniin, Ellagsäure und Brevifolincarbonsäure und gegebenenfalls das von Corilagin und Geranniin verschiedene Ellagitannin umfasst.

15. Verwendung nach einem der Ansprüche 1 bis 14, wobei die Gallotannine in dem Arzneimittel in einer Konzentration von 0.1 bis 99.9 % formuliert sind.

16. Verwendung nach einem der Ansprüche 1 bis 15, wobei das Extrakt oder die Fraktion in einer Konzentration von 0.1 bis 99.9 % in dem Arzneimittel vorliegt.

17. Verwendung nach einem der Ansprüche 1 bis 16, wobei Phyllanthus Phyllanthus amarus ist.

## Revendications

1. Utilisation d'un extrait de *Phyllanthus* ou d'une fraction de celui-ci ou d'un gallotannin ou d'une combinaison de gallotannins pour la préparation d'une composition pharmaceutique pour la prévention ou le traitement de maladies reliées à un rétrovirus associées à un rétrovirus résistant à un inhibiteur nucléosidique et/ou un rétrovirus résistant à un inhibiteur non-nucléosidique.

2. Utilisation selon la revendication 1, dans laquelle les maladies sont traitées par inhibition de la réplication d'un rétrovirus résistant à un inhibiteur nucléosidique et/ou d'un rétrovirus résistant à un inhibiteur non-nucléosidique.

3. Utilisation selon la revendication 1, dans laquelle les maladies sont traitées par inhibition de la capture d'un rétrovirus résistant à un inhibiteur nucléosidique et/ou d'un rétrovirus résistant à un inhibiteur non-nucléosidique.

4. Utilisation selon l'une quelconque des revendications 1 à 3, dans laquelle ledit rétrovirus est un VIH.

5. Utilisation selon la revendication 4, dans laquelle ledit VIH est un VIH-1.

6. Utilisation selon l'une quelconque des revendications 1 à 3, dans laquelle ledit extrait est un extrait aqueux, un extrait alcoolique, un extrait par eau/alcool, un extrait par l'hexane ou un extrait par le CO₂.

7. Utilisation selon la revendication 6, dans laquelle ledit alcool est le méthanol ou l'éthanol.

8. Utilisation selon l'une quelconque des revendications 1 à 5, dans laquelle ladite fraction est une fraction de protéines ou une fraction de gallotannins.

9. Utilisation selon l'une quelconque des revendications 1 à 5 ou 8, dans laquelle ledit gallotannin est, ou ladite fraction de gallotannins comprend, la corilagine.

10. Utilisation selon l'une quelconque des revendications 1 à 5 ou 8, dans laquelle ledit gallotannin est, ou ladite fraction de gallotannins comprend, la géraniine.

11. Utilisation selon l'une quelconque des revendications 1 à 5 ou 8, dans laquelle ledit gallotannin est, ou ladite fraction de gallotannins comprend, l'acide ellagique.

12. Utilisation selon l'une quelconque des revendications 1 à 5 ou 8, dans laquelle ledit gallotannin est, ou ladite fraction de gallotannins comprend, l'acide brévifoline carboxylique.

13. Utilisation selon l'une quelconque des revendications 1 à 5 ou 8, dans laquelle ledit gallotannin est, ou ladite fraction de gallotannins comprend, un ellagitannin différent de la corilagine, de la géraniine, de l'acide ellagique ou de l'acide brévifoline carboxylique.

14. Utilisation selon l'une quelconque des revendications 1 à 5 ou 8, dans laquelle ladite combinaison de gallotannins ou ladite fraction de gallotannins comprend au moins deux parmi la corilagine, la géraniine, l'acide ellagique et l'acide brévifoline carboxylique et, de façon optionnelle ledit ellagitannin différent de la corilagine et de la géraniine.

15. Utilisation selon l'une quelconque des revendications 1 à 14, dans laquelle lesdits gallotannins sont formulés dans ladite composition pharmaceutique en une concentration de 0,1 à 99,9%.

16. Utilisation selon l'une quelconque des revendications 1 à 15, dans laquelle ledit extrait ou ladite fraction est présent dans ladite composition pharmaceutique en une concentration de 0,1 à 99,9%.

17. Utilisation selon l'une quelconque des revendications 1 à 16, dans laquelle ledit *Phyllanthus* est le *Phyllanthus amarus.*
